(19) Europäisches Patentamt / European Patent Office / Office européen des brevets

(11) **EP 1 292 395 B1**

(12) **EUROPEAN PATENT SPECIFICATION**

(45) Date of publication and mention
of the grant of the patent:
**30.01.2008 Bulletin 2008/05**

(51) Int Cl.:
**B05B 1/00** (2006.01)

(21) Application number: **01910482.7**

(22) Date of filing: **08.02.2001**

(86) International application number:
**PCT/US2001/004158**

(87) International publication number:
**WO 2001/058508 (16.08.2001 Gazette 2001/33)**

(54) **ACTUATOR NOZZLE FOR METERED DOSE INHALER**

BETÄTIGUNGSDÜSE EINER DOSIERAEROSOLE

BUSE D'ACTIONNEUR POUR AEROSOL-DOSEUR

(84) Designated Contracting States:
**AT BE CH CY DE DK ES FI FR GB GR IE IT LI LU MC NL PT SE TR**

(30) Priority: **09.02.2000 GB 0002798**

(43) Date of publication of application:
**19.03.2003 Bulletin 2003/12**

(73) Proprietor: **GLAXO GROUP LIMITED**
**Greenford, Middlesex UB6 ONN (GB)**

(72) Inventor: **ZHAO, Junguo**
**Research Triangle Park, NC 27709 (US)**

(74) Representative: **Giddings, Peter John et al**
**GlaxoSmithKline**
**Corporate Intellectual Property (CN925.1)**
**980 Great West Road**
**Brentford,**
**Middlesex TW8 9GS (GB)**

(56) References cited:
**EP-A- 0 412 524        GB-A- 2 276 101**
**US-A- 4 074 861        US-A- 4 322 037**
**US-A- 4 647 013        US-A- 5 497 944**

**Description**

FIELD OF THE INVENTION

The present invention relates to nozzles for aerosol propellant systems, and more particularly, to aerosolization spray nozzles for metered dose inhalers.

BACKGROUND OF THE INVENTION

Medicaments, broadly including therapeutic, prophylactic and diagnostic agents, may be delivered locally to the lung or systemically through the lung for the treatment, prophylaxis or diagnosis of illnesses and other conditions. Many devices are used to deliver medicaments to the lung, including metered dose inhalers (MDIs). MDIs are aerosol delivery systems having a reservoir of compressed, low boiling point liquid formulated with a medicament. MDIs are designed to meter a predetermined quantity of the medicament formulation and dispense the dose as an inhalable particulate cloud.

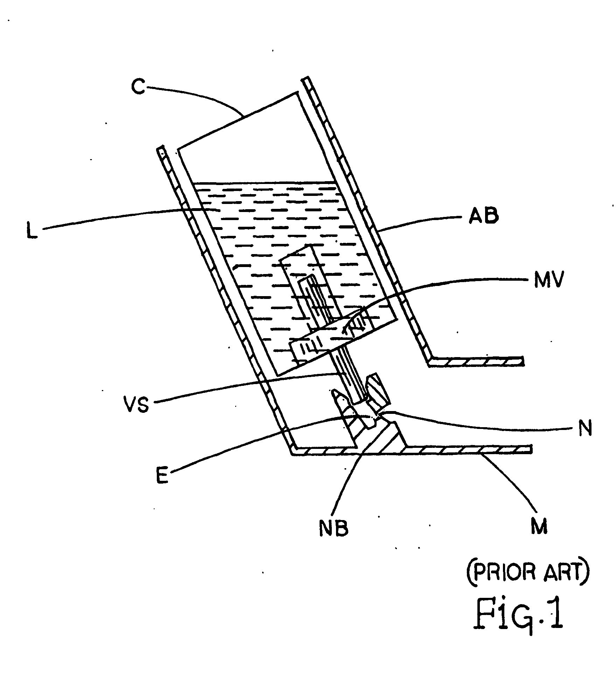

A typical commercially available MDI is disclosed in US Pat. No. 5,031,610. As depicted in FIG. 1, an MDI includes an actuator body **AB** in which is positioned a canister **C.** Canister **C** contains a liquid medicament **L** in solution or suspension with a low boiling point propellant. The most common propellants include the chlorofluorocarbons, p-11 and p-12, and the fluorocarbons, p-134a or p-227, alternative propellants may include carbon dioxide, all of which are gaseous at room temperature and standard atmospheric pressure.

The canister possesses a metering valve **MV** for measuring discrete doses of the drug formulation fluid. A valve stem **VS** extends from the metering valve and acts as a conduit to pass the metered dose into a nozzle block **NB** situated in the actuator body, in which the valve stem is seated.

The nozzle block has a passageway extending through it that forms an internal chamber **E** in which the propellant formulation expands. As a result of manufacturing and design, chamber E typically contains ancillary internal cavities and surface features. A nozzle channel **N,** which is aligned with a mouthpiece opening **M,** exits the expansion chamber **E,** approximately tangential to the longitudinal axis of the axis of the valve stem.

Typically, the nozzle channel has a diameter that is about 0.5 mm and a length (measured between the inside to the chamber **E** and the outer surface of the nozzle block) which is approximately 1.5 mm.

To use this type of MDI, the patient places the mouthpiece against the lips and actuates the MDI by depressing the canister into the actuator. Upon actuation, a metered dose is measured by the valve and is expelled from the valve stem. The expelled dose passes into and through the internal expansion chamber **E** of the nozzle block and exits the nozzle block from the nozzle channel **N.** A patient inhales through the mouthpiece upon the release of the metered dose and inhales the drug dose as it exits the inhaler.

It is appreciated by those skilled in the art that medicaments delivered through inhalation devices optimally target specific sites in the pulmonary system. These sites include the nasal passages, the throat, and various locations within the lung, such as the bronchi, bronchioles and alveolar regions. The ability to deliver drugs to a target area is largely dependent on the size of the medicament particle, its velocity and settling properties. Particles having an aerodynamic diameter less than 2 microns are considered to be optimal for deposition in the alveolar region of the lung. Particles that have an aerodynamic diameter of between 2 and approximately 5 microns tend to be more suitable for delivery to the bronchiole or bronchi regions. Particles with an aerodynamic size range greater than 6 microns are suitable for delivery to the laryngeal region, throat or nasal passages.

As used herein, particles of six microns or less are referred to as "respirable" or "within the respirable range." In turn, the percentage of the particles within a given dose of aerosolized medicament that is of "respirable" size, as compared to the total dose, is referred to as the "fine particle fraction" (FPF) or "fine particle mass" (FPM) of the dose.

Fine particle fraction and the deposition behavior of inhaled solution or suspension based medicaments are greatly dependent on the construction and performance of the delivery system device. Generally speaking, FPF increases with decreasing diameter of the actuator nozzle exit orifice. *See,* Lewis, et al, "Effects of Actuator Orifice Diameter on Beclomethasone Dipropionate Delivery From a PMDI HFA Solution Formulation ", Poster Presented at Respiratory Drug Delivery VI (4-7 May 1998), Hilton Head South Carolina, USA.

This phenomenon may be explained as a result of a decrease in the velocity of the particle being ejected from the nozzle exit orifice. A reduction in the size of propellant droplets or particles exiting from an MDI nozzle significantly decreases the velocity of ejected material. By reducing the size and velocity of particles exiting an MDI nozzle before the particles reach a patient's throat, it is possible to minimize throat deposition, thus, increase the fine particle fraction of the delivered dose. One way of reducing both the size and the velocity of ejected fluid droplets in MDIs is to decrease the exit orifice diameters in the nozzle channels.

Device manufacturers have realized this relationship and have made devices accordingly. Bespak (United Kingdom) manufactures an MDI actuator, Model No. BK633, having a nozzle with a .25 mm orifice diameter, with reported high fine particle doses. 3M (Minneapolis, MN USA) manufacture the Proventil® MDI having a nozzle exit diameter of approximately .3 mm diameter, also with purported high FPF.

It has been observed that such small diameter nozzles, however, experience device clogging due to material deposition. Material deposition may lead to device failure, which can be life threatening in cases where the MDI is used in emergency treatment, such as treating an acute asthma attack. Because small diameter exit orifices results in the deposition of material in a MDIs expansion chamber, a balance is generally required between the ability of the device to generate appropriately sized particles and the ability of the device to avoid clogging. This balance leads to relatively larger nozzle orifice diameters being used, such as approximately .5mm.

Because experience has taught that nozzle openings of the type shown in Figure 1 clog more easily as orifice size falls significantly below .5 mm in diameter, it is typical that a nozzle orifice will be in the order of 0.5 mm in diameter, and the respirable fraction of the plumes emitted from such devices is generally on the order of 15 to 40 percent depending on the formulations and testing methods.

The respirable fraction of the aerosol plume emitted from an MDI may be increased by the use of an add-on, called a large volume spacer, which is attached to the MDI mouthpiece. A typical spacer has a holding chamber of 250 and 1000 ml in size which can hold the MDI aerosol plume before the patient inhales. A spacer can effectively slow down the MDI aerosol plume and allow more time for the aerosol droplet to dry before inhalation. As a result, the patient will inhale a dryer and softer aerosol plume with much less drug deposition in the mouth and throat. In addition, the spacer allows greater flexibility in synchronizing triggering the MDI and patient inhalation.

Due to impaction deposition and gravitational settlement inside the spacer, however, the drug loss in a spacer is significant. As a result, the dose leaving the spacer is much smaller than the dose emitted from an MDI without a spacer. The net effect is that a spacer effectively shifts the drug deposition from patient's mouth and throat to the spacer, but without significant improvement in the net amount of drugs delivered to the targets.

Thus, while spacers have proven to be useful in terms of percentage of respirable particles exiting the device, they suffer from the disadvantage of not actually increasing to a significant degree the amount of respirable material entering the lung.

Further disadvantages to spacers are that the spacer adds to the cost and overall size of an MDI. Obviously, increased cost places a larger financial burden on those paying for health care. Increased size makes the MDI more inconvenient to carry and makes the patient more conspicuous when they use their inhaler in public. Patient compliance requires the device to be carried and used. Larger devices are less likely to be carried, and a large device may bring undesirable attention to its user in public. Hence, it is generally desirable to make MDIs as small as possible, and avoiding the use of a spacer is one way to accomplish this end.

In light of the above, there is a perceived need to develop a MDI capable of generating particle plumes containing particles of respirable size.

There is a need to develop a MDI that is capable of generating high fine particle fractions.

There is a need to develop a MDI device that reduces device deposition and/or throat deposition.

There is a need to develop a MDI that reduces the velocity and/or size of particles/droplets being emitted from the device.

There is a need to develop a MDI that avoids device failure due to clogging.

Lastly, there is a need to develop a MDI that is small and inconspicuous, and avoids the need for a spacer.

The novel inhaler design described herein is intended to address one or more of these considerations, as well as other which are appreciated by one of ordinary skill in the art.

As background art there may be mentioned US-A-4 322 037, US-A-5 497 944, US-A-4 074 861 and EP-A- 0 412 524.

## SUMMARY OF THE INVENTION

The applicants have surprisingly found that the increased degree of material deposition typically encountered with the use of nozzle orifices having a diameter of .3mm or less may be reduced to a level at or below that experienced with larger diameter nozzles while still producing the high fine particle fractions achievable through using small diameter orifice nozzles (.3 mm or less). This is accomplished by limiting the length of the portion of the nozzle channel which is . 3 mm or less in diameter to .5 mm or less in length

Such a nozzle configuration, optionally, embodied in a selected smooth, low adhesion, or low surface energy material, with or without other design characteristics, yields a novel MDI actuator having superior performance characteristics to those disclosed in the prior art.

Thus, the present invention is a metered dose inhaler according to claim 1.

The other claims set out optional features of the present invention.

Hence, it is an object of the present invention to provide a novel MDI as disclosed and/or claimed herein.

It is another object of the present invention to provide a MDI actuator that is capable of generating medicament particles of respirable size.

Additionally, it is an alternative object of the present invention to provide a MDI actuator that yields doses of high fine particle fraction.

Further, it is an alternative object of the present invention to provide a MDI actuator that generates a particle plume of acceptable velocity, thus decreasing the amount of throat deposition.

Further still, it is an alternative object of the invention to provide a MDI actuator that does not need a spacer.

Lastly, it is an alternative object of the present invention to provide an inhaler with decreased incidence of nozzle clogging and associated device failure.

DESCRIPTION OF THE DRAWINGS

Figure 1 is a cross sectional view of the previously described prior art MDI.

Figures 2A-2D depict various alternative channel configurations in the actuator nozzle of the present invention.

Figures 3A depicts an additional alternative nozzle configurations in the actuator nozzle of the present invention.

Figure 3B depicts a enlarged portion of the nozzle of 3A.

Figures 4A depicts an additional alternative nozzle configurations in the actuator nozzle of the present invention.

Figure 4B depicts a enlarged portion of the nozzle of 4A.

Figure 5 is a sectional representation of a MDI incorporating an actuator nozzle of the present invention.

Figure 6 depicts the MDI of Figure 5, with an exploded view of the novel actuator.

Figure 7 is a diagram of a conical nozzle.

Figure 8 is a graphical representation of the data shown in Table 2.

Figure 9 is a graphical representation of the deposition data of Tables 5A and 5B.

DETAILED DESCRIPTION OF THE INVENTION

Preferred Embodiments

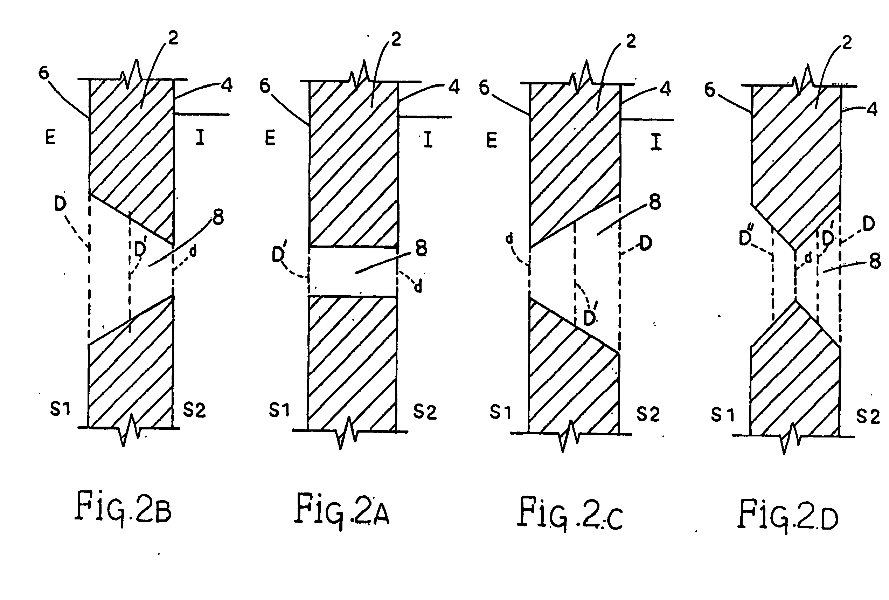

A first embodiment of the nozzle of the actuator of the present invention is shown in various configurations in Figures 2A- 2D. As shown therein, nozzle **2** comprises a plate having a first surface **4** and a second surface **6,** with an exit channel **8** extending between these two surfaces.

The applicants have found that the design and dimensions of the channel, alone or in conjunction with the surface characteristics of the nozzle, yield improved performance characteristics in devices employing such nozzles.

In particular, it has been found that nozzles having channels with minimal diameters of 0.3 mm or less may be employed in MDI actuators to yield high respirable fractions of aerosolized particles. Such high respirable fractions are obtainable without experiencing the same degree of clogging as experienced in prior art small diameter nozzle MDIs. The length of that portion of the nozzle channel which has a diameter of 0.3 mm or less (also referred to herein as "the narrow portion of the channel") is no more than 0.5 mm in length.

Figure 2A shows a first embodiment of such a nozzle. In Figure 2A, channel **8** is shown as parallel sided and tubular. The channel has a diameter **d** of 0.3mm or less, and the total length of the channel, in this case the distance between first and second surfaces **4** and **6,** is 0.5 mm or less.

Figure 2B depicts a second embodiment of a nozzle of the present invention, wherein channel **8** is tapered and the diameter of the channel as measured at first surface **4** is smaller than the diameter of the channel as measured at second surface **6.** At some point along the length of the channel, the diameter of the channel reaches a diameter **D',** where the diameter of the channel is 0.3mm. High respirable fractions have been produced when the length of the channel which is 0.3 mm or less, in this case the length of the channel between **D'** and **d**, is 0.5 mm or less. The point within the narrow portion of the channel at which the diameter is at its minimum is referred to herein as the "constriction" of the channel. In Figure 2B, the constriction is at the point of minimal channel diameter **d.**

Figure 2C depicts a further embodiment of the nozzle of the present invention wherein the taper of the channel is the reverse of that shown in 2B, such that the larger diameter **D** is measured at first surface **4** and the smaller diameter **d** is measured at second surface **6.** Again **D'** represents the point where the diameter of the channel is 0.3 mm, and the length between **D'** and the constriction **d** is 0.5 mm or less.

Figure 2D represents a still further embodiment of the nozzle of the present invention. In Figure 2D, the nozzle channel has a convergent taper, wherein channel **8** extends between first surface **4** and second surface **6,** and tapers inwards from both of these surfaces. The tapered walls of the channel meet at a constriction, shown with diameter **d.** **D'** and **D''** represent points along the length of the channel where the diameter of channel is 0.3 mm or less. The distance between **D'** and **D''** is ideally 0.5 mm or less.

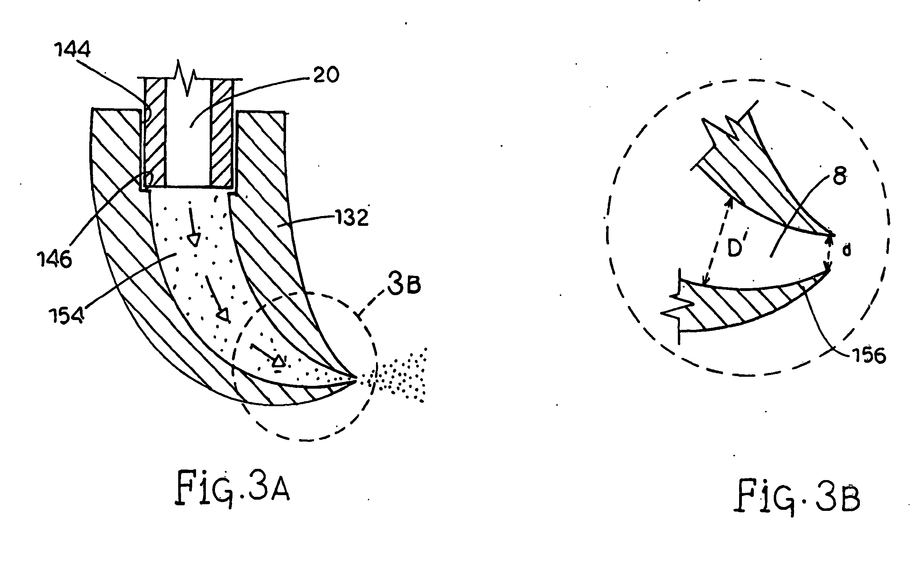

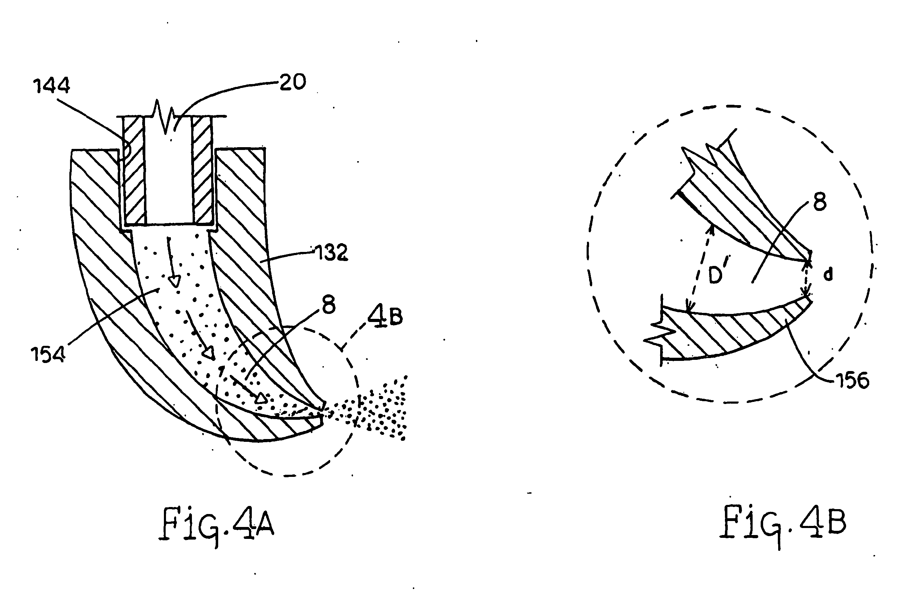

In any embodiment having a channel where the channel diameter at some point is 0.3 mm or less and the channel diameter differs along its length, the portion of the channel which is larger than 0.3 mm is not considered to play a significant role in yielding high fine particle fractions. Hence, as shown in Figures 2 B, 2C and 2D, for example, the portion of the channel between **D** and **D'** could be of any length. Application of the feature, is represented in Figures 3A and 3B and 4A and 4B, which depict nozzle configurations of the instant invention where the channel **8** is a continuous extension of a passageway defined within in a nozzle block **132**.

Figure 3B is an enlarged section of 3A, whereas Figure 4B is an enlarged section of Figure. 4A. Figures 3B and 4B show the narrowing of channel **8** as it approaches a small channel diameter (constriction) **d.** The ultimate exit of the channel (indicated at **d**) is 0.3 mm or less in diameter. As was the case of other embodiments of the invention, the channel may have narrow portion prior to **d** where the diameter of the channel reaches 0.3 mm, shown as **D'**. The distance between **d** and **D'** is 0.5 mm or less.

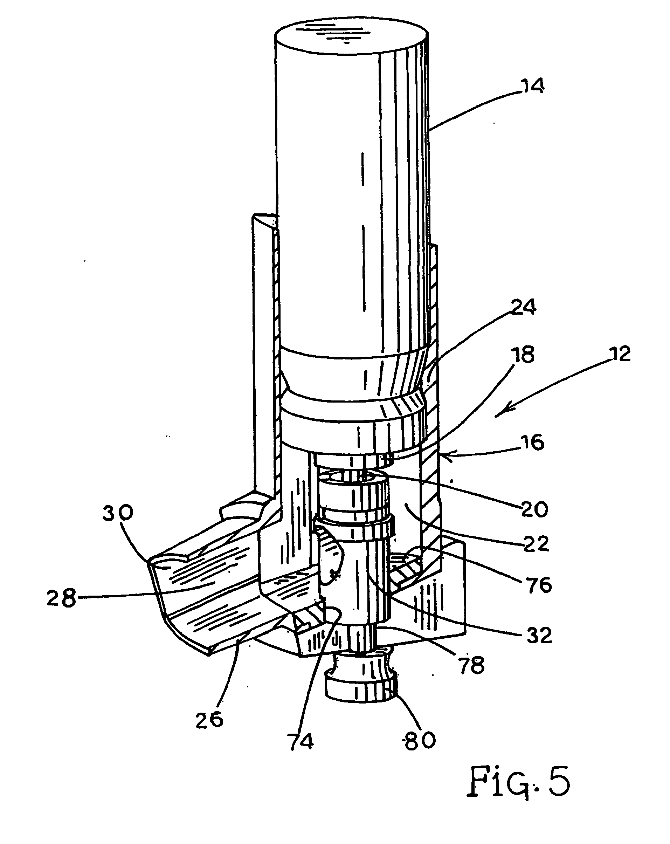

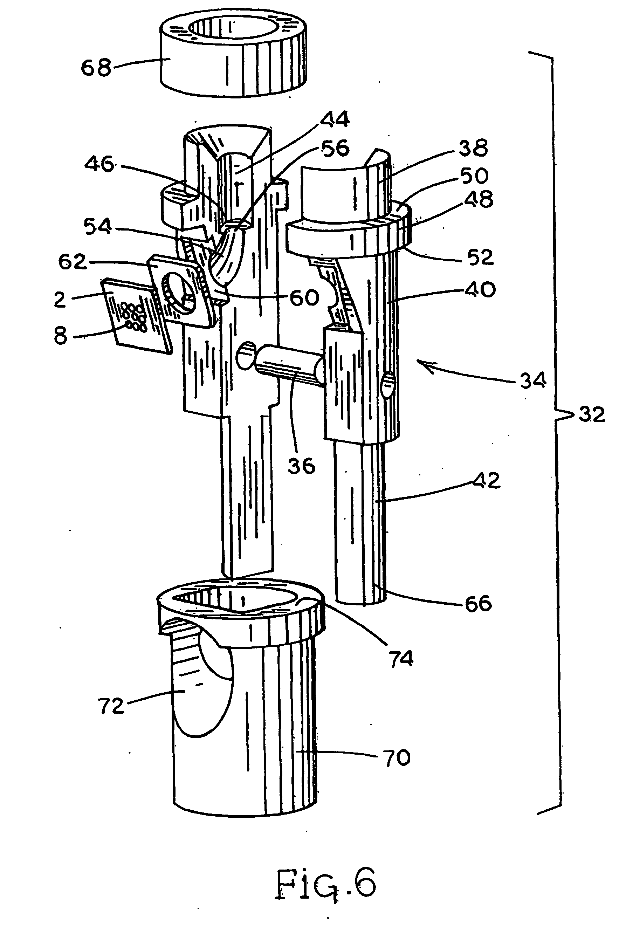

The nozzle channel configurations are employed in various actuator nozzle block designs for incorporation into MDI actuators. The group of embodiments shown in Figures 2A - 2D may be employed in an MDI as shown in Figures 5 and 6.

Figure 5 depicts an inhaler **12** that comprises a canister **14** and an actuator body **16**. Canister **14** contains fluid **F** and possesses a metering valve assembly **18** having a valve stem **20** extending therefrom.

The metering valve assembly operates longitudinally to cause the metering and release of a quantity of the fluid into the valve stem **20** which possesses an internal conduit (not shown) of a predetermined volume. Thus, the metered dose passes through the valve stem conduit to exit the canister.

Canister **14** is positioned within a central cavity **22** formed within the main body **24** of actuator body **16**. A mouthpiece **26** extends from main body **24** and defines a passageway **28** having a mouthpiece opening **30**.

A nozzle block **32** is positioned within central cavity **22**. The nozzle block may be an integrally formed system that is made by any suitable process known to the art, for example by injection molding and subsequent processing. Alternatively, the nozzle block may be a single component or may be made of multiple components.

As shown in Figure 6, nozzle block **32** is of the multiple component variety. It is composed of a primary actuator component **34** having two halves coupled by a connector pin **36**. The primary actuator component has an upper extension **38**, a middle portion **40**, and a lower extension **42**.

The upper extension **38** has a valve stem recess **44** defined within it and the valve stem recess has a floor **46**. Between the upper extension and lower extension is positioned a shoulder **48**, which defines an upper abutment surface **50** and a lower abutment surface **52**.

The middle portion **40** of the primary actuator component **34** defines an internal chamber **54**. Internal chamber **54** has a proximal and distal portion. The proximal portion defines an inlet **56**, formed in floor **46** of valve stem recess **44**. The distal portion of the chamber has an outlet formed in a wall of the chamber, which comprises a nozzle **2**.

As will be appreciated, this portion of the wall of the nozzle block which forms the nozzle could be formed integrally with the remainder of the nozzle block, or could be a separately manufactured piece which is then assembled with the remainder of the nozzle block. In the embodiment shown in Figure 6, the wall of the nozzle block is composed of a separate plate or film of material. The structure of this plate or film is discussed herein with reference to Figures 2A-2D. Nozzle plate **2** is fitted into a distal recess **60** in the middle portion of the primary actuator component. A washer or gasket **62** is positioned between the internal surface of the plate and the distal recess in a sealed fashion.

Plate **2** has an internal surface facing the chamber and an external surface facing in the opposite direction from the internal surface, with channel(s) **8** extending therethrough.

The primary actuator component **34** is assembled by coupling the two halves of the component together with pin **36**. Tubular upper collar **68** is frictionally fitted over the upper extension **38** of the nozzle plate holder, until it contacts the upper abutment surface **50** of shoulder **48**. A second tubular collar, or lower collar, **70** fits over stem **66** and is disposed over the middle portion **40** of the primary actuator component. When assembled, the upper end **74** of the lower collar abuts the lower abutment surface **52** of shoulder **48**. The lower collar **70** has a tapered generally tubular exit port **72** that extends through the collar. The lower collar is formed such that its internal configuration conforms to the exterior surface angling of the middle portion of the primary actuator component with which it makes physical contact. When assembled, the tapered exit port of the lower collar and outlet the nozzle are aligned. The tubular collar acts to hold the assembled nozzle block together.

So configured, the nozzle block **32**, which in this case refers to the collective assembly of the primary actuator component, pin, washer, nozzle (barrier) plate and collars, is positioned with the central cavity of the actuator body, as shown in FIG. 5. Nozzle block **32** is ideally fitted within a receiving recess **74** formed in the floor **76** of the main body **24** of actuator body **16**. When so positioned, the tapered port **72** of the nozzle block is aligned with passageway **28** of mouthpiece **26**. Stem **66**, which may be threaded, extends through a hole **78** in the floor of the actuator body and is fastened with fastener **80**, to secure the actuator in this position.

To assemble the metered dose inhaler depicted in FIG. 5, canister **14** is positioned in central cavity **22**, such that valve stem **20** is received within the valve stem recess or stem block **44** of nozzle block **32**. The end of the valve stem abuts the floor **46** of the valve stem recess, and a seal is formed between the external surface of the valve stem and the walls of the valve stem recess. This seal may result from friction fitting between the stem and recess surfaces but may also be enhanced with the assistance of suitable materials or coatings applied to the recess walls and/or outer stem surface.

When the valve stem is positioned in this fashion, the open end of the valve stem conduit is aligned with the inlet opening in the floor of the valve stem recess that extends into the internal chamber **54** of the nozzle block. The valve stem internal conduit, the chamber inlet, the chamber, and the chamber outlet are in fluid communication with each other, and thus comprise a fluid flow pathway through the nozzle block.

The valve stem conduit and chamber make up the volume of an expansion chamber within the inhaler, which will be discussed in detail later herein.

Alternative nozzle block designs are shown again with reference to in Figures 3A and 3B and 4A and 4B. In these embodiments, the nozzle block **132** is formed in a single piece. The nozzle block has a valve stem recess **144** having a floor **146.** The floor **146** defines a chamber inlet that extends into an internal chamber **154.** At the other end of the chamber is a chamber outlet, which takes the form of a channel **8** extending through the nozzle block.

As will be appreciated by those of ordinary skill, the nozzle block is intended to be positioned within the central cavity of an actuator body, as is described with reference to FIG. 5. The channel and the opening of the mouthpiece of the actuator body are generally aligned, such that material ejected from the channel is directed through the mouthpiece opening.

When assembled in a metered dose inhaler, the valve stem recess **144** of the nozzle block engages the valve stem **20** extending from the canister (not shown). The open end of the valve stem contacts the floor **146** of the nozzle block, in the same fashion as previously described.

To use this device, the canister is depressed into the actuator. The motion of the canister causes the metering valve to a meter a fixed volume of the fluid forming an individual dose. The metered dose of the fluid passes into and through the valve stem conduit and into the internal chamber of the nozzle block through the chamber inlet. Upon leaving the pressurized environment of the canister, the propellant component of the fluid rapidly expands within the expansion chamber and is expelled as individual droplets or particles through the outlet on the nozzle block.

### Channel Configuration

As explained previously, the performance of a metered dose inhaler in creating particles of respirable size from a metered dose of fluid without clogging is influenced by the dimensions of exit channel **8**. Particle size generation and associated fine particle mass is perceived to be a function of small channel diameter. To take advantage of this, the instant application achieves high FPF production by incorporating small diameter channel(s) in the nozzle. These channels have a diameter of 0.3 mm or less, preferably between 0.25 and 0.05 mm, such as 0.2 mm, 0.15 mm, or 0.1 mm.

The deposition of material on the internal and external surfaces of the device reduces the amount of material that actually gets delivered to the targeted areas in a patient's body. Material deposition in an MDI may occur within the expansion chamber of the actuator nozzle block, for example on the internal walls of the expansion chamber, including within the outlet channel(s), and on the exterior surface of the nozzle in the vicinity of the nozzle channel exit(s). When the material is deposited in standard nozzles found in the prior art, it tends to accumulate within the expansion chamber and on the exterior surface of the nozzle. The material buildup can produce material bridging which eventually chokes off flow through the nozzle channel.

It is the applicants' observation that material buildup is most likely to cause problems in clogging when it occurs within the internal chamber of the nozzle block, especially in that portion of the channel where the channel diameter falls below 0.3 mm. Thus, the applicants have surprisingly found that the clogging normally associated with small diameter valves may be reduced or avoided by decreasing the length of that portion of the channel which has a diameter of 0.3 mm or less (the "narrow portion") to 0.5 mm or less in length. For example, the narrow portion of the channel may be less than 0.5mm, preferably 0.4 mm or less, such as 0.35 nun, 0.3 mm, 0.25 mm, 0.2 mm, 0.15mm, 0.1 mm or .05 mm, as well as smaller lengths. These smaller lengths can be infinitesimal, such as when a tapered channel nozzle is 0.3 mm at its smallest point. In such a case, the length of the narrow portion would be only the thickness of the rim of the channel at its exterior surface.

The channels themselves may be parallel-sided, as in 2A, tapered, as in 2B, 2C, 3A and 4A, or convergent as in Fig 2D. The tapered form may be with a constriction of the channel at any point along its length. The preferred channel profile has tapered sides where the constricted constriction is at the exit of the channel (i.e., a forward tapered channel). It has been observed that this forward tapered channel works synergistically with a reduced thickness nozzle plate to further reduce deposition and minimize nozzle clogging.

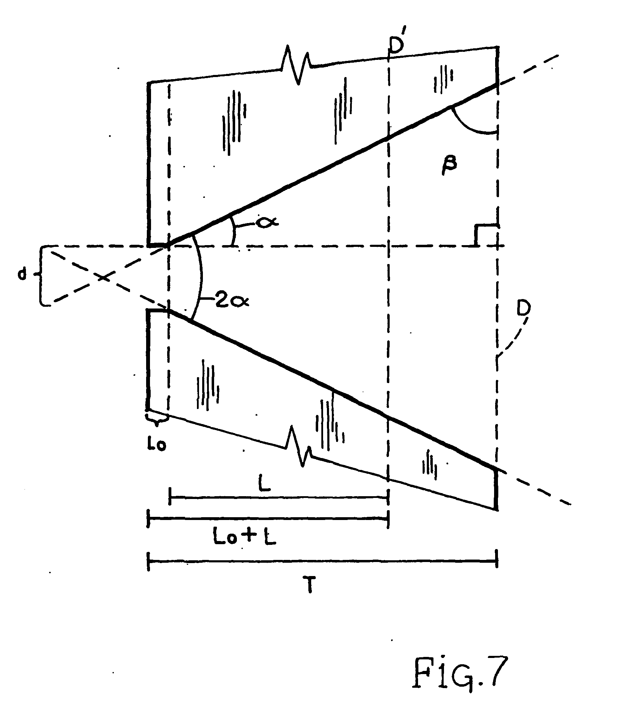

With reference to FIG. 7, the angle of the taper β of the channel can be any that is capable of being achieved in manufacturing. The angle of taper in the tapered form is preferably between 30 and 60 degrees. In some cases, the taper angle is determined by the material and manufacturing process selected. For, example, photolithographed silicon has an angle of taper of 54.7 degrees consistently, due to the chemical process employed and the properties of the material subjected to that process.

The channels can have any cross-sectional configuration including circular, oval, square, rectangular, or poly-angular, which can be achieved in manufacturing. The cross-sectional shape of the channel may also depend on the material selected. For example, chemically etched photolithographed silicon generally has channels with a square cross-section. Preferably, the channels are circular in cross section. Suitable processes for creating channels include chemical etching, mechanical, thermal or laser drilling, molding or machining.

Diameter measurements for the instant invention are taken on a side-to-side fashion for channels with circular cross-sections. When channels are non-circular in cross-section, the diameter is determined by hydraulic diameter, which is defined as 4x the cross-sectional area divided by the perimeter at the cross section. As an example, the hydraulic diameter for a square = $4d^2/(4d)$, where d is the length of the square side.

Any number of exit channels can be included in the nozzle block, however, the preferred number of channels is 16 or less, more preferably 9 or less, and most preferably from 1 to 4 channels.

The channels may be positioned in any suitable arrangement about the nozzle plate or selected portion of the nozzle block wall. They may be at any suitable distance from each other. An appropriate channel arrangement is one which balances the structural integrity and robustness of the walled portion, the potential of material to be deposited on a surface of a nozzle, and the achievable fine particle mass of the plume emitted from the actuator. To maximize robustness and to optimize fine particle fraction, the holes should be as far apart as possible. To minimize interior surface deposition, however, the holes should be kept as close as possible. With nozzles employing multiple tapered channels, the robustness of the walled portion is largely determined by the distance between the larger openings on a particular surface of the portion or the wall defining the channel (or plate). It is believed that a configuration where the larger tapered channel openings are spaced approximately 0.05 mm apart is optimal for minimizing deposition while providing sufficient rigidity and fine particle fraction performance. It will be appreciated that, depending on the material selected, the minimum distances between the large orifices are heavily influenced by manufacturing tolerance of the material selected to make the nozzle.

The number of channels and the diameter of the channels should be configured such that the total minimum cross sectional flow area within the channels at the constriction is less that $0.25mm^2$, and preferably less than $0.2 mm^2$, and most preferably $0.1 mm^2$ or less.

Preferably, the cross sectional flow area is such that the delivery time of the metered dose is 2 seconds or less, and preferably 1 second or less, with the most preferred delivery time being 0.5 seconds or less.

## Materials of Construction

The portion of the nozzle block defining the exit channel(s) may be an integral component of the nozzle block formed at the same time as the nozzle block, or may be a separate component of the nozzle block which is assembled to form a completed nozzle block unit. In either case, the plate or portion of the wall containing the nozzle channel(s) may be made of the same material or a different material than the remainder of the nozzle block. Moreover, the nozzle block and/or that portion of the nozzle block defining the channel may be constructed of any suitable material that can withstand the pressures encountered with a desired propellant system.

The portion of the nozzle block wall defining the channels ("walled portion") and/or nozzle block, preferably are fabricated with a surface which has properties which reduce the likelihood of material deposition. Such characteristics may be imparted by materials or surface finishes which are smooth, and/or non-adhesive and/or low surface energy. Such materials may include, but are not limited to metals, such as stainless steel, gold, nickel, brass and aluminum; silicon; or various polymeric materials.

The polymeric materials include polyethyhlene (PE), polypropylene (PP), polymethylmethylacrylate (PMMA), polyvinyl chloride (PVC), polyvinyldiene chloride (PVDC), polyvinyl fluoride (PVF), polyvinyldiene fluoride (PVDF), polychlorotrifluoroethylene (PCTFE), polytetrafluoroethylene (PTFE), fluorinated ethylene propylene (FEP), perfluroroalkoxy (PFA), polyamide (PA), polyethylene terephthalate (PET), polybutylene terephthalate (PBT), polyetherimide (PEI), polyamideimide (PAI), polyimide (PI), polysulfone (PS), polyarylsulfone (PAS) polyethersulfone (PES), polyphenylene sulfide (PPS), polyetheretherketone (PEEK), polydimethylsiloxane (PDMS) and polycarbonate (PC) or combinations and blends thereof.

These polymeric materials are available from typical suppliers such as DuPont, Dow, General Electric, ICI, 3M, Monsanto, Amoco, BASF, Allied Signal, Bayer, Eastman, Phillips, LNP etc.

Materials may also be of composite structure, comprising of a base substrate and layer coating the substrate. The base substrate material may comprise of any of the aforementioned materials, or any other material known to the art which would be deemed suitable for the purposes contemplated herein. The coating layer may include a fluoropolymer, silicone or fluorosilicone based material or other material or material blend with low adhesion properties, or which is smooth, or which posses low surface energy.

The materials may be a pure material or may be a blended material, such as blended polymeric materials. Alternatively, multiple layers of coating materials may be sequentially applied onto the base material.

The performance of the nozzle block (and nozzle) may be further enhanced by constructing one or more of the components of the nozzle block from, or coating such component with, a material which has a low surface energy, is smooth and/or which has low adhesive properties.

Typical low surface energy materials include fluoropolymer and silicone materials such as PTFE, FEP, PFA, PVDF and PDMS. These materials have surface energies in the range of 18 to 25 dynes/cm. The material may, itself posses such properties, or may be coated to possess such properties. Plasma coating may be used to impart fluoropolymers, silicones, or other low surface energy coatings to the material so treated.

The above coatings can be applied through any coating/manufacturing process known in the industry, including spray coating, dip coating, electrostatic coating, chemical vapor deposition, plasma enhanced chemical vapor deposition, cold plasma deposition and laminating.

An alternative characteristic, smoothness of the surface, which is also believed to impact particle adhesion of the surface, may be an inherent feature of a material or may be imparted by subsequent processing. Smooth surfaces on the nozzle reduce drug deposition and/or the tendency of the nozzle to clog. The smoothness properties for a given material may be tested by standard testing apparatuses known to the art. For example, testing of smoothness can be done with a instrument known as a Pocket Surf II® portable surface roughness gauge (Federal Products Co, Providence, RI, USA). The gauge uses a probe with a stylus traveling along the testing surface and measures the surface roughness. Two parameters may be used for comparison. The first is $R_a$, an average surface roughness value, calculated as a root mean square height of roughness irregularities measured from a mean line, within a fixed length. The $R_a$ range of the instrument is 0.03 - 6.35 $\mu$m. The second parameter, $R_{max}$, is the maximum roughness depth within the evaluation length. The instrument range is 0.2-25.3 $\mu$m.

In testing of various materials, the stylus is allowed to travel a distance of 5 mm on a sample of a material used to construct a given walled portion. To standardize the procedure, the instrument was first calibrated with a standard surface of 3.02 micrometer roughness. Then individual surfaces were tested giving the following results:

Table 1: Surface Roughness of Various Materials.

| Material | Ra ($\mu$m) | Rmax ($\mu$m) | Travel length | Comments |
|---|---|---|---|---|
| Plastic actuator | .28 | 3.0 | 5 | |
| Micro-machined stainless Steel | 0.6 | 3.7 | 5 | |
| Micro-machined brass | 0.25-0.97 | 2.4-4.8 | 5 | |
| 0.002" stainless steel film | 0.12 | 0.8 | 5 | |
| Silicon wafer | <0.03* | <0.2* | 5 | |
| *$R_a$ and $R_{max}$ were at or below the detection level for the instrument | | | | |

Silicon wafers that are typically prepared for integrated circuit applications are very smooth and possess low adhesion properties. Thus, silicon and stainless steel are preferred materials for the walled portion of the present device.

There are additional considerations that also effect the performance of metered dose inhalers other than channel diameter and length and material selection. For example, the design of the fluid flow path through the nozzle block acts to effect the device's performance.

**Expansion Chamber Volume**

Improved device performance may also be contributed to through design of the expansion chamber and the fluid flow path that extends through the nozzle block, in areas other than the channel configuration. In the present invention, the internal void of the valve stem conduit and the actuator's internal chamber collectively form an expansion chamber volume. The expansion chamber volume is thus the total internal volume of these two components. In the embodiment depicted in Figures 3A, 4A, 5 and 6, the volume of this expansion chamber is 30 microliters or less, preferably 20 microliters or less, more preferably between 5 and 20 microliters, such as 18 microliters. It is believed that deposition within the nozzle block is reduced by incorporating a smaller expansion chamber volume.

**Expansion Chamber Flow Properties**

The fluid flow from the inlet to the internal chamber to the outlet is designed to be fluid dynamic, in that the internal construction and materials of the nozzle block are designed to reduce the thickness of the boundary layer of the viscous fluid coming into contact with the inner surfaces of the nozzle block, and to minimize contact area of the flowing fluid with the internal surfaces. Reduction of the boundary layer thickness serves to minimize material accumulation on the internal surfaces of the expansion chamber during normal use of the device. This is accomplished by minimizing the surface roughness of the chamber, by incorporating gradual changes in the geometry of the internal components and by selecting appropriate internal surface contouring.

Optimally, the internal chamber extends between the chamber inlet and the nozzle without forming any abrupt angles or ancillary cavities, such as those seen in the prior art example shown in FIG. 1. It is appreciated by the inventors that features which create dead space in the chamber provide deposition sites for material flowing therethrough. Thus, the present invention also relates to an internal chamber design having a tubular, smooth-sided configuration that non-abruptly curves from the inlet to the outlet of the chamber so to reduce fluid resistance within the internal chamber. So configured, the chamber directs the material flowing through chamber directly toward the portion of the wall defining the exit channel, thus reducing material being directed to areas not experiencing some degree of fluid flow where deposition would be likely to occur.

The elimination of such deposition sites significantly reduces the amount of material build-up experienced during normal use of the device and yields higher material output from the nozzle. More importantly, it reduces carry-over effect (in which medicament from a previous dose is delivered in a subsequent dose), and therefore increases product performance consistency. In Figures 3A and 4A, this curved tubular expansion chamber decreases in diameter from the inlet to the outlet, whereas in FIG. 6, the tubular expansion chamber, 56, gently curves toward the barrier plate defining nozzle 2. In either form, the barrier layer is minimized and the amount of dead space is reduced through elimination of ancillary cavities.

### External Features of the Nozzle

Further reduction of material deposition and likelihood of clogging may be achieved through modification of the external surface features of the nozzle where the exit channels emerge from the nozzle block. As shown in FIGs. 3B and 4B, the channel outlet forms a tip or protrusion where it emerges on the external surface of the nozzle block. In FIG. 3B, the tip comes to a sharp, conical, external point. This feature is believed to be capable of reducing potential deposition by decreasing the surface area immediately adjacent the channel exit, thus reducing the area at which deposition would occur. The protrusion so acts to create a more focused aerosol plume. In FIG. 4B, the tip **156** is modified to have a slightly flattened appearance, which is intended to serve the same functions and achieve the same beneficial result as the tip shown in FIG. 3B.

METHOD OF OPERATION

As briefly described above, and with reference to FIG. 5, a patient may assemble the MDI of the present invention by inserting the canister into the actuator body of the inhaler. The valve stem of the canister is engaged in the valve stem recess (which is alternatively depicted in FIGs. 3A, 4A or 5). So assembled, the patient places the mouthpiece of the inhaler to the lips and triggers the release of a dose of medicament.

Once triggered, a metered dose of fluid is delivered from the actuator through the conduit of the valve stem, through the chamber inlet and into the internal chamber of the actuator. Once released from the highly pressurized content of an MDI canister, the propellant rapidly expands in the expansion chamber defined by the internal volume of the valve stem and the internal volume of the chamber. The expanded material flows, due to the fluid-dynamic design of the nozzle block, toward the exit channel(s) of the chamber. At this point, the material is driven through the exit channel and the ejected material is aerosolized as a plume containing droplets or particles of respirable size.

The patient, by appropriately synchronizing the inhalation effort with the triggering of the device, causes the ejected plume to be carried into their pulmonary system, thus, accomplishing local or systemic delivery of the medicament.

Synchronization of inhalation and release of the dosage can also be assisted by including a breath actuator component on the MDI. The breath actuator may be a movable vane, a diaphragm, a pressure triggered valve, or a breath actuated electrical switch responsive to the pressure drop associated with inhalation. Such devices are commonly known in the art.

Thus, the disclosed device permits the practice of a method (the method itself not forming part of the invention) for generating a particle cloud from an actuator nozzle comprising the steps of :

1. providing an actuator for a metered dose inhaler comprising: a nozzle block having a fluid flow path extending therethrough, said fluid flow path defined by an internal chamber having an inlet and an outlet; said outlet being defined in a portion of said nozzle block and comprising an exit channel extending therethrough; wherein said exit channel has a narrow portion wherein the diameter of the channel is 0.3 mm or less, said narrow portion being 0.5 mm or less in length; said narrow portion optionally including a constriction, said constriction having a diameter of less than .3 mm;

2. introducing a metered dose of fluid containing a low boiling point propellant into said inlet; and

3. expelling said fluid from said outlet in particulate form.

The invention also allows practice of a method (the method itself not forming part of the invention) for the treatment, prophylaxis or diagnosis of a condition of disease in a patient comprising:

1. providing a metered dose inhaler comprising:

a. a canister containing a medicament in suspension or solution with a low boiling point fluid propellant, said canister possessing a metering valve for metering a dose of said suspension or solution, and hollow valve stem for transferring said metered dose from said metering valve, and

b. an actuator comprising: a nozzle block having a fluid flow path extending therethrough, said fluid flow path defined by an internal chamber having an inlet and an outlet; said outlet being defined in a portion of said nozzle block and comprising an exit channel extending therethrough; wherein said exit channel has a narrow portion wherein the diameter of the channel is 0.3 mm or less, said narrow portion being 0.5 mm or less in length; said narrow portion optionally including a constriction, said constriction having a diameter of less than 0.3 mm; and

2. introducing a metered dose of fluid into said inlet;

3. ejecting said metered dose from said outlet in particulate form; and

4. delivering said ejected metered dose to the pulmonary system of the patient.

## EXPERIMENTATION CONCERNING FINE PARTICLE FRACTION

### EXPERIMENT 1

The device used to generate the data in Table 2 is generally described above in reference to Figures 5, 6 and 2C. The device was equipped with a cylindrical mouthpiece with walled portion made of a silicon plate 0.4 millimeters thick. The nozzle had a single tapered channel (the channel inlet being larger than its outlet) 0.2mm in diameter and square in profile.

The device was tested through cascade impaction against a commercial Ventolin HFA® Metered Dose Inhaler actuator (sold in Europe by Glaxo Wellcome) using a standard albuterol sulfate/134a formulation, which may be described as being represented by the description attributed to FIG. 1.

The cascade impactor testing apparatus was a 1 ACFM non-viable 8-stage cascade impactor (Anderson Instruments, Inc., Smyrna, GA, USA). Two versions of the cascade impactor were used. The first had a typical cascade impaction throat (identified herein as a "long throat"). The second had a modified shortened throat, where the length of the horizontal part of the throat was one half of the original long throat length. It is believed that the modified short form gives a better comparison to in-vivo performance.

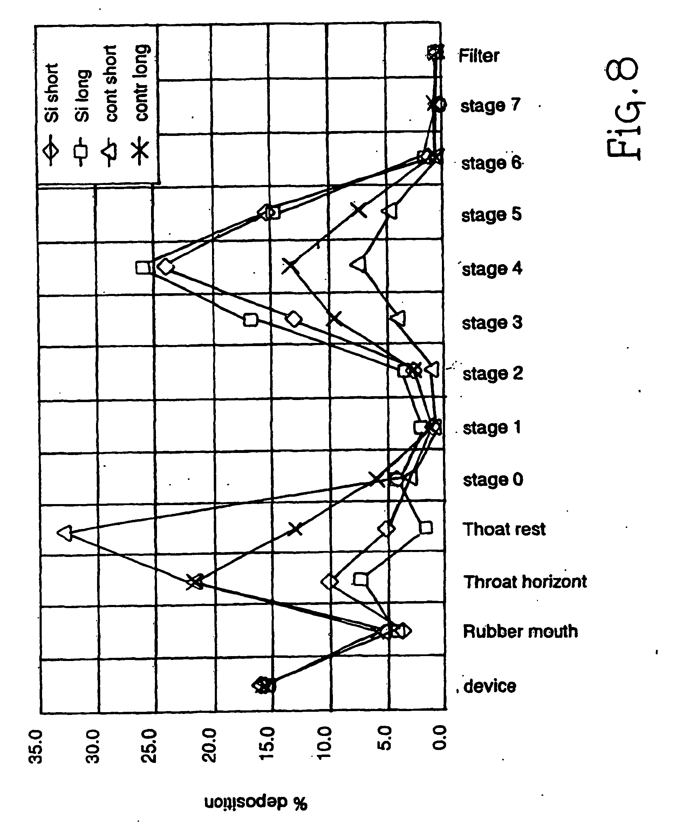

The data presented here is a typical representation of the new device performance vs. a regular commercial actuator. Typically, particles/droplets deposited on stages 2-6 is considered respirable, and is an indicator for drug delivery efficiency. It is believed that the results obtained with the short throat are a better indicator of the drug delivery efficiency. This data is graphically represented in Figure 8.

Table 2: Normalized Drug Distribution In MDI Cascade Impaction Test

| Normalized | Silicon short | Silicon long | Control short | Control long | volume aerodynamic diameter, $\mu$m | In-vivo deposition |
|---|---|---|---|---|---|---|
| Device | 16.0 | 15.4 | 15.4 | 15.6 | >10.0 | Oral and throat deposition |
| Rubber mouth | 4.2 | 4.5 | 5.5 | 6.1 | | |
| Throat horizontal | 10.1 | 7.3 | 22.2 | 22.0 | | |
| Throat rest | 5.3 | 2.1 | 32.9 | 13.2 | | |
| Stage 0 | 4.1 | 4.6 | 2.9 | 6.2 | 9.0-10.0 | |
| Stage 1 | 1.8 | 2.3 | 1.0 | 1.9 | 5.8-9.0 | |
| Stage 2 | 2.9 | 3.9 | 1.2 | 2.5 | 4.7-5.8 | Pharynx |
| Stage 3 | 13.3 | 16.6 | 4.4 | 9.5 | 3.3-4.7 | Trachea & primary bronchi |
| Stage 4 | 24.0 | 25.9 | 7.4 | 13.4 | 2.1-3.3 | Secondary bronchi |
| Stage 5 | 15.5 | 14.7 | 4.9 | 7.2 | 1.1-2.1 | Terminal bronchi |
| Stage 6 | 1.8 | 1.7 | 0.7 | 0.9 | 0.65-1.1 | Alveoli |
| Stage 7 | 0.3 | 0.3 | 0.8 | 0.8 | 0.43-0.65 | Alveoli |
| Stage F | 0.7 | 0.8 | 0.6 | 0.7 | <0.43 | |
| Dose | 100.0 | 100.0 | 100.0 | 100.0 | | |
| Actuator | 16.0 | 15.4 | 15.4 | 15.6 | | |
| Throat + rubber | 19.6 | 13.9 | 60.6 | 41.3 | | |
| Throat | 15.4 | 9.4 | 55.1 | 35.2 | | |
| Sum 2-6 | 57.6 | 62.7 | 18.7 | 33.5 | | |

The data shown in Table 2 demonstrate that the present invention significantly reduces deposition in the throat area and significantly increases the amount of material deposited in stages 2-6 of the impactor compared to control.

EXPERIMENT 2

Table 3 provides cascade impaction data showing increased fine particle fraction with the nozzle design of the instant invention as compared to control. All were tested with pressurized albuterol sulfate / 134a canisters. The control again was a standard, commercially available polypropylene (PP) Ventolin HFA® MDI with a straight, cylindrical nozzle, similar to that shown in Figure 1. The tested silicon (Si) nozzle had a square, tapered nozzle channel shape, with the larger opening on the interior surface of the walled portion, as depicted in Figure 2C. The tested stainless steel (SS) nozzle had a generally cylindrical, parallel-sided nozzle channel shape, as depicted in Figure 2A. All nozzles had a single exit channel. Data represent the percentage of material in stages 2-6 of the Anderson Cascade Impactor.

Table 3: Fine Particle Mass As A Percentage Of Total Emitted Dose

| Throat | Nozzle material | Nozzle diameter mm | Nozzle length mm | Run 1 | Run 2 | Run 3 | Mean |
|---|---|---|---|---|---|---|---|
| Short | PP (control) | 0.5 | 1.5 | 20.62 | 21.24 | 18.66 | 20.17 |
| | Si | 0.2 | 0.4 | 54.04 | 54.80 | 57.58 | 55.47 |
| | SS | 0.15 | 0.1 | 48.42 | 43.22 | | 45.82 |
| | | 0.23 | 0.15 | 57.06 | 46.85 | | 51.96 |
| | | 0.24 | 0.1 | 44.64 | 40.92 | | 42.78 |
| Long | PP (control) | 0.5 | 1.5 | 40.44 | 33.44 | 33.54 | 35.81 |
| | Si | 0.2 | 0.4 | 66.49 | 53.20 | 62.72 | 60.80 |
| | SS | 0.15 | 0.1 | 48.86 | 29.75* | | 39.31 |
| | | 0.23 | 0.15 | 59.43 | 60.19 | | 59.81 |
| | | 0.24 | 0.1 | 53.87 | 55.49. | | 54.68 |
| * excessive throat deposition, due to misalignment of actuation orientation. | | | | | | | |

EXPERIMENT 3

Table 4 represents cascade impaction studies done on actuators using formulations of salmeterol hydroxynapthoate in 134a propellant. Studies were conducted using MDI canisters providing 25 $\mu$g salmeterol per actuation, and volumes to provide 120 actuations. The fine particle fraction in this case is the sum of cascade impaction data collected for material in stages 3-5 as a percentage of total actual dose. The nozzles tested were all single nozzle channel varieties. The control actuator nozzle was a standard actuator from a Flovent HFA® MDI commercially available from Glaxo Wellcome in Europe, generally represented in Figure 1.

Test actuator nozzles included a square channeled, tapered silicon (Si) actuator nozzle (as in FIG. 2C); a cylindrical channeled stainless steel (SS) nozzle as in FIG. 2A) and; a cylindrical channel polyimide nozzle (as in FIG. 2A).

The polyimide nozzle was very flexible and deformed upon mounting on the nozzle block, making it very difficult to control the plume exit orientation and causing more mouthpiece and throat deposition. Dimensions for all tested nozzles are indicated in Table 4.

Table 4: Normalized Drug Distribution in MDI Cascade Impaction Test

| CI throat | Long | | | Short | | |
|---|---|---|---|---|---|---|
| Actuator material | Control | SS | Si | Control | Polyimide | Si |
| Nozzle diameter, mm | 0.5 | 0.23 | 0.2 | 0.5 | 0.2 | 0.2 |
| Nozzle length, mm | 1.5 | 0.15 | 0.4 | 1.5 | 0.13 | 0.4 |
| Device | 15.5 | 15.1 | 17.6 | 15.3 | 25.1 | 18.1 |
| Throat | 34.6 | 18.3 | 13.8 | 58.3 | 21.0 | 21.4 |
| Stage 0 | 8.2 | 8.1 | 6.9 | 4.1 | 7.7 | 6.4 |
| Stage 1 | 2.7 | 3.0 | 3.3 | 1.6 | 2.6 | 2.8 |
| Stage 2 | 2.7 | 3.7 | 4.8 | 1.4 | 2.9 | 5.0 |
| Stage 3 | 7.2 | 12.3 | 13.1 | 3.5 | 8.4 | 11.0 |
| Stage 4 | 14.6 | 21.5 | 22.7 | 7.0 | 16.2 | 18.9 |
| Stage 5 | 12.2 | 15.0 | 15.4 | 7.0 | 13.3 | 13.7 |
| Stage 6 | 1.8 | 2.2 | 2.1 | 1.2 | 2.2 | 2.0 |
| Stage 7 | 0.5 | 0.6 | 0.6 | 0.4 | 0.6 | 0.5 |
| Filter | 0.2 | 0.2 | 0.1 | 0.1 | 0.1 | 0.1 |
| Total | 100.0 | 100.0 | 100.0 | 100.0 | 100.0 | 100.0 |

(continued)

| CI throat | Long | | | Short | | |
|---|---|---|---|---|---|---|
| Total Ex-Device | 84.5 | 84.9 | 82.4 | 84.7 | 74.9 | 81.9 |
| FPM% | 34.1 | 49.0 | 51.3 | 17.7 | 38.2 | 43.5 |

**EXPERIMENTATION CONCERNING DRUG DEPOSITION**

<u>EXPERIMENT 4</u>

Drug deposition in the expansion chamber of an MDI is related to the likelihood of actuator clogging or dose inconsistency. Accumulated drug material in the expansion chamber may fall off to clog the atomization nozzle, or be released upon subsequent actuation to yield wide variation in the amount of active material delivered to a patient from one actuation of an MDI to another. Thus, the less the drug deposits in the expansion chamber, the better for MDI performance.

In Tables 5A and 5B, the drug deposition in the expansion chamber is averaged over 20 actuations comparing various nozzle configurations. Actuators 1-5 of the present invention shown in FIG 5A have diameters, below .3 mm, and narrow portions less than 5mm in length. Significantly less clogging is seen for these short nozzle channel actuators than with actuators having long channel lengths, as shown in Actuators 7-9 in Table 5B. The deposition figures for these short nozzle length actuators are less than or equivalent to the results seen in standard large diameter channel MDI actuators, as represented by Actuator 6 in Table 4B

The expansion chamber (including some nozzles) for the first 5 actuators, was micromachined and the surface finishes were not as smooth as the last 4 injection molded ones. Even so, and with smaller nozzles, the first 5 had less drug deposition in the expansion chamber, indicating the importance of nozzle length and shape.

Table 5A. Drug deposition in expansion chamber

| Actuator | Material | Shape | Diam. (mm) | Total length of channel (mm) | Expansion chamber, % of total dose |
|---|---|---|---|---|---|
| 1. | Stainless Steel | Straight | 0.22 | 0.15 | 7.73 |
| 2. | Stainless Steel | Straight | 0.25 | 0.2 | 8.15 |
| 3. | Aluminum | Conical ($\alpha$=21°) | 0.22 | 0.7* | 9.29 |
| 4. | Stainless Steel | Straight | 0.25 | 0.1 | 9.31 |
| 5 | Silicon | Conical ($\alpha$= 35.3°) | 0.2 | 0.4* | 10.05 |

* The conical nozzle shown in FIG. 7 has a thickness **T.** The length of the conical portion of the nozzle which has a diameter of 0.3 mm or less may be calculated by the equation:

$$D' = d + (2L \times \tan \alpha) \qquad L = \frac{D'-d}{2 \times \tan \alpha}$$

Wherein **D'** is the point where the diameter of the channel is 0.3 mm; **d** is the measure of the smaller diameter of the channel at the point it becomes conical; **L** is the length between **d** and **D'**; and angle $\alpha$ is the half-cone angle (i.e., ½ the full cone angle 2 $\alpha$, or the complementary angle to the angle of taper, β) of the tapered channel.

In the case of Actuator 3 in Table 5A, **D'** = 0.3mm, d = 0.22 mm, a = 21 degrees (the total cone angle, 2$\alpha$, being 42 degrees), and hence the length, L, of the conical portion of the channel which has a diameter below .3mm is 0.104 mm.

To calculate the total length of the channel which is .3 mm or less, the short parallel-sided constriction portion, $L_o$ ,of the channel of Actuator No. 3 must be added. In this case $L_o$ was approximately 0.1mm in length, so the total nozzle length having a diameter below 0.3 mm is calculated as $L + L_o$ = 0.2 mm.

Actuator 5 in Table 5A was also conical, but because the total length of the channel was less than .5mm, (i.e., 0.4 mm) and is within the desirable range of narrow portion lengths, and therefore, no calculation is included herein.

Table 5B. Drug deposition in expansion chamber in non-thin walled actuator nozzles

| Actuator | Material | Shape | Diam. (mm) | Total length of channel (mm) | Expansion chamber, % of total dose |
|---|---|---|---|---|---|
| 6. | Inj.-Molded Plastic (control) | Straight | 0.5 | 1.5 | 10.28 |
| 7. | Inj.-Molded Plastic (BK633) | Straight | 0.25 | 0.6 | 15.50 |
| 8. | Inj.-Molded Plastic (Proventil®) | Straight | 0.3 | 0.7 | 16.31 |
| 9. | Inj.-Molded Plastic (E149) | Straight | 0.3 | 0.9 | 16.60 |

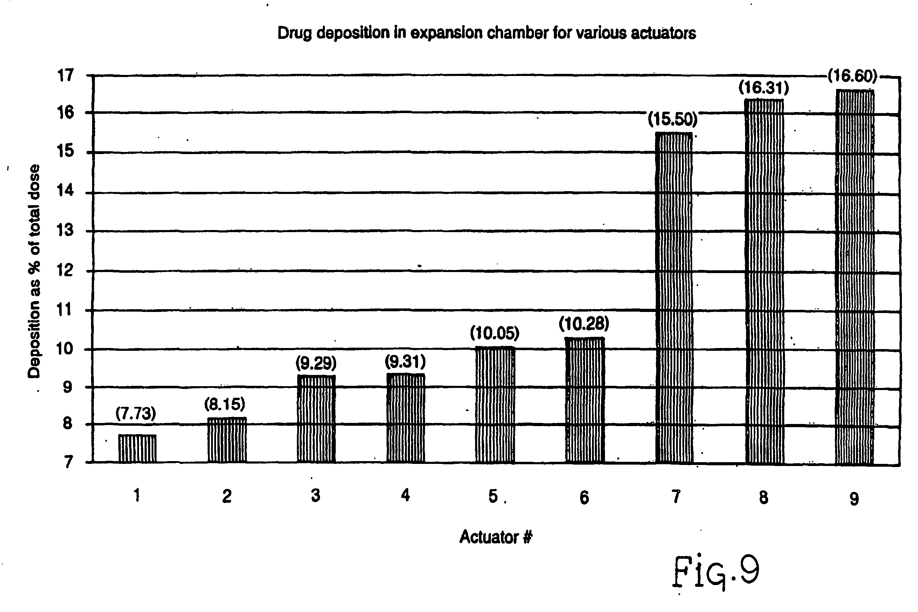

Figure 9 is a graphical representation of the deposition data shown in Tables 5A and 5B, showing the significant decrease in clogging behavior associated with small diameter, short narrow portion actuator nozzles.

**Claims**

1.  A metered dose inhaler (12) comprising:

    a canister (14) containing a fluid (F) comprising a low boiling point propellant formulated with a medicament;
    a metering valve (18) connected to said canister,
    a hollow valve stem (20) extending from said metering valve, the hollow of said valve stem having an internal valve stem volume and being movable between extended and retracted positions relative to the canister for metering the fluid from the canister; and
    an actuator (16) in which the canister is locatable comprising:

    a nozzle block (32) for receipt of the valve stem to enable the valve stem to move between its extended and retracted positions relative to the canister, the nozzle block having a recess (44; 144), with a floor (46; 146), into which the valve stem is insertable, and a fluid flow path extending therethrough, said fluid flow path defined by an internal chamber (54; 154) having an inlet (56) defined in the recess floor and an outlet, said outlet being defined in a portion of said nozzle block and comprising an exit channel (8) extending therethrough, wherein said exit channel has a narrow portion, the narrow portion having a diameter which is 0.3 mm or less and a length which is 0.5 mm or less, and
    a mouthpiece (26), said mouthpiece having a passageway (28), and said passageway being aligned with said exit channel, such that fluid metered from said canister, passes through said valve stem, passes into said fluid flow path, exits said exit channel, and is ejected from said passageway of said mouthpiece.

2.  The inhaler of claim 1, wherein said portion of said nozzle block defining said exit channel comprises a plate (2) having said exit channel extending therethrough.

3.  The inhaler of claim 1, wherein said portion of said nozzle block defining said exit channel comprises a surface comprising a material, wherein said material has a surface energy of 25 dynes/cm or less.

4.  The inhaler of claim 1, wherein said portion of said nozzle block defining said exit channel comprises a surface comprising a material, wherein said material has a smooth surface having a $R_a$ value of 1 micron or less.

5.  The inhaler of claim 1, wherein said portion of said nozzle block defining said exit channel comprises a crystalline, metal or a polymeric material.

6.  The inhaler of claim 5 wherein said metal is selected from the group consisting aluminum, gold, nickel, and stainless steel.

7.  The inhaler of claim 5, wherein said material comprises a polymeric material and said polymeric material comprises polyethyhlene (PE), polypropylene (PP), polymethylmethylacrylate (PMMA), polyvinyl chloride (PVC), polyvinyldiene chloride (PVDC), polyvinyl fluoride (PVF), polyvinyldiene fluoride (PVDF), polychlorotrifluoroethylene (PCTFE), polytctrafluoethylene (PTFE), fluorinated ethylene propylene (FEP), perfluroroalkoxy (PFA), polyamide (PA), polyethylene terephthalate (PET), polybutylene terephthalate (PBT), polyetherimide (PEI), polyamideimide (PAI), polyimide (PI), polysulfone (PS), polyarylsulfone (PAS) polyethersulfone (PES), polyphenylene sulfide (PPS), polyetheretherketone (PEEK), polydimethylsiloxane (PDMS), and polycarbonate (PC) or blends thereof.

8. The inhaler of claim 1, wherein said exit channel is conical, and the narrow portion includes a construction the diameter of which is less than 0.3 mm and greater than about 0.05 mm.

9. The inhaler of Claim 1, wherein said narrow portion is 0.4 mm or less in length.

10. The inhaler of claim 2, wherein said plate has a thickness of 1.0 millimeter or less.

11. The inhaler of Claim 10, wherein said plate from 1.0 millimeter to 0.05 millimeters.

12. The inhaler of claim 1, wherein said fluid flow path has a volume of 30 microliters or less.

13. The inhaler of Claim 1, wherein said internal chamber is tubular and conical, and the diameter of the chamber decreases between its inlet and outlet.

14. The inhaler of claim 13, wherein said internal chamber curves between said inlet and outlet.

15. The inhaler of claim 1, wherein the nozzle block defines a protrusion, and said exit channel emerges from said block from said protrusion.

16. The inhaler of claim 15, wherein said protrusion forms an acute point where said channel emerges from said protrusion.

17. The inhaler of claim 15, wherein said protrusion comprises a blunt tip where said channel emerges from said protrusion.

18. The inhaler of Claim 1, wherein said internal chamber is smooth and conical and lacks surface features or ancillary cavities between the inlet and outlet which would create dead space therein.

19. The inhaler of Claim 1 or 2, wherein said nozzle block possesses between 1 and 9 exit channels.

20. The inhaler of claim 5 wherein said crystalline material is silicon.

**Patentansprüche**

1. Abmeßdosis-Inhalator (12), umfassend:

einen Behälter (14) enthaltend ein Fluid (F), umfassend ein niedrigsiedendes Treibmittel, das mit einem Medikament formuliert ist;
ein Abmeßventil (18), das mit dem Behälter verbunden ist;
einen hohlen Ventilschaft (20), der sich vom Meßventil aus erstreckt, wobei der Hohlraum des Ventilschafts ein inneres Ventilschaftvolumen aufweist und zwischen ausgefahrenen und zurückgezogenen Positionen relativ zum Behälter beweglich ist, zum Abmessen des Fluids aus dem Behälter; und
ein Betätigungsglied (16), worin der Behälter lokalisierbar ist, umfassend:

einen Düsenblock (32) zur Aufnahme des Ventilschafts, damit sich der Ventilschaft zwischen seinen ausgefahrenen und zurückgezogenen Positionen relativ zum Behälter bewegen kann, wobei der Düsenblock eine Vertiefung aufweist (44, 144) mit einem Boden (46, 146), in die der Ventilschaft inserierbar ist, und einen Fluidflußweg, der sich dadurch erstreckt, wobei der Fluidflußweg durch eine innere Kammer (54, 154) definiert ist mit einem Einlaß (56), definiert in dem Vertiefungsboden, und einem Auslaß, wobei der Auslaß in einem Teil des Düsenblocks definiert ist, und umfassend einen Ausgangskanal (8), der sich dadurch erstreckt, wobei der Ausgangskanal einen engen Bereich aufweist, wobei der enge Bereich einen Durchmesser aufweist, der 0,3 mm oder weniger beträgt, und eine Länge, die 0,5 mm oder weniger beträgt, und

ein Mundstück (26), wobei das Mundstück eine Passage (28) aufweist und wobei die Passage mit dem Ausgangskanal ausgerichtet ist, so daß das Fluid, das aus dem Behälter abgemessen wird, durch den Ventilschaft passiert, in den Fluidflußweg passiert, aus dem Ausgangskanal austritt und von der Passage des Mundstücks ausgestoßen wird.

2. Inhalator gemäß Anspruch 1, wobei der Teil des Düsenblocks, der den Ausgangskanal definiert, eine Platte (2) aufweist, durch die sich der Ausgangskanal erstreckt.

3. Inhalator gemäß Anspruch 1, wobei der Teil des Düsenblocks, der den Ausgangskanal definiert, eine Oberfläche umfaßt, umfassend ein Material, wobei das Material eine Oberflächenenergie von 25 Dynes/cm oder weniger aufweist.

4. Inhalator gemäß Anspruch 1, wobei der Teil des Düsenblocks, der den Ausgangskanal definiert, eine Oberfläche umfaßt, umfassend ein Material, wobei das Material eine glatte Oberfläche mit einem Ra-Wert von 1 μm oder weniger aufweist.

5. Inhalator gemäß Anspruch 1, wobei der Teil des Düsenblocks, der den Ausgangskanal definiert, ein kristallines, metallisches oder polymeres Material umfaßt.

6. Inhalator gemäß Anspruch 5, wobei das Metall ausgewählt ist aus der Gruppe bestehend aus Aluminium, Gold, Nickel und rostfreiem Stahl.

7. Inhalator gemäß Anspruch 5, wobei das Material ein polymeres Material umfaßt, und wobei das polymere Material Polyethylen (PE), Polypropylen (PP), Polymethylmethacrylat (PMMA), Polyvinylchlorid (PVC), Polyvinyldienchlorid (PVDC), Polyvinylfluorid (PVF), Polyvinyldienfluorid (PVDF), Polychlortrifluorethylen (PCTFE), Polytetrafluorethylen (PTFE), fluoriertes Ethylenpropylen (FEP), Perfluoralkoxy (PFA), Polyamid (PA), Polyethylenterephthalat (PET), Polybutylenterephthalat (PBT), Polyetherimid (PEI), Polyamidimid (PAI), Polyimid (PI), Polysulfon (PS), Polyarylsulfon (PAS), Polyethersulfon (PES), Polyphenylensulfid (PPS), Polyetheretherketon (PEEK), Polydimethylsiloxan (PDMS) und Polycarbonat (PC) oder Mischungen davon umfaßt.

8. Inhalator gemäß Anspruch 1, wobei der Ausgangskanal konisch ist und der enge Bereich eine Konstriktion aufweist, deren Durchmesser weniger als 0,3 mm und mehr als ungefähr 0,05 mm beträgt.

9. Inhalator gemäß Anspruch 1, wobei der enge Bereich eine Länge von 0,4 mm oder weniger aufweist.

10. Inhalator gemäß Anspruch 2, wobei die Platte eine Dicke von 1,0 mm oder weniger aufweist.

11. Inhalator gemäß Anspruch 10, wobei die Platte eine Dicke von 1,0 mm bis 0,05 mm aufweist.

12. Inhalator gemäß Anspruch 1, wobei der Fluidflußweg ein Volumen von 30 Mikroliter oder weniger aufweist.

13. Inhalator gemäß Anspruch 1, wobei die innere Kammer röhrenförmig und konisch ist und wobei der Durchmesser der Kammer sich zwischen Einlaß und Auslaß vermindert.

14. Inhalator gemäß Anspruch 13, wobei die innere Kammer sich zwischen Einlaß und Auslaß krümmt.

15. Inhalator gemäß Anspruch 1, wobei der Düsenblock einen Vorsprung definiert und wobei der Ausgangskanal aus dem Block von dem Vorsprung austritt.

16. Inhalator gemäß Anspruch 15, wobei der Vorsprung einen spitzen Punkt bildet, wobei der Kanal aus dem Vorsprung austritt.

17. Inhalator gemäß Anspruch 15, wobei der Vorsprung eine stumpfe Spitze umfaßt, wobei der Kanal aus dem Vorsprung austritt.

18. Inhalator gemäß Anspruch 1, wobei die innere Kammer glatt und konisch ist und ihr Oberflächenmerkmale oder zusätzliche Höhlungen zwischen Einlaß und Auslaß fehlen, die einen Totraum darin erzeugen würden.

**19.** Inhalator gemäß Anspruch 1 oder 2, wobei der Düsenblock zwischen 1 und 9 Austrittskanäle besitzt.

**20.** Inhalator gemäß Anspruch 5, wobei das kristalline Material Silizium ist.

**Revendications**

**1.** Inhalateur doseur (12) comprenant :

un boîtier (14) contenant un fluide (F) comprenant un agent propulseur à bas point d'ébullition formulé avec un médicament ;
une valve doseuse (18) raccordée audit boîtier ;
une tige de valve creuse (20) s'étendant à partir de ladite valve doseuse, le creux de ladite tige de valve comportant un volume interne de tige de valve et étant mobile entre des positions étendue et rétractée par rapport au boîtier pour doser le fluide à partir du boîtier ; et
un actionneur (16) dans lequel le boîtier peut être logé comprenant :

un bloc buse (32) destiné à la réception de la tige de valve afin de permettre que la tige de valve se déplace entre ses positions étendue et rétractée par rapport au boîtier, le bloc buse comportant un renfoncement (44 ; 144), avec un fond (46 ; 146), dans lequel la tige de valve peut être insérée, et un trajet d'écoulement de fluide s'étendant à travers celle-ci, ledit trajet d'écoulement de fluide étant défini par une chambre interne (54 ; 154) comportant une admission (56) définie dans le fond du renfoncement et une évacuation, ladite évacuation étant définie dans une partie dudit bloc buse et comprenant un canal de sortie (8) s'étendant à travers celui-ci, ledit canal de sortie comportant une partie étroite, la partie étroite ayant un diamètre mesurant 0,3 mm ou moins et une longueur mesurant 0,5 mm ou moins, et
un embout buccal (26), ledit embout buccal comportant un passage (28), et ledit passage étant aligné avec ledit canal de sortie, de telle sorte que le fluide dosé à partir dudit boîtier passe par ladite tige de valve, passe dans ledit trajet d'écoulement de fluide, sort dudit canal de sortie et est éjecté à partir dudit passage dudit embout buccal.

**2.** Inhalateur selon la revendication 1, dans lequel ladite partie dudit bloc buse définissant ledit canal de sortie comprend une plaque (2) comportant ledit canal de sortie s'étendant par celui-ci.

**3.** Inhalateur selon la revendication 1, dans lequel ladite partie dudit bloc buse définissant ledit canal de sortie comprend une surface comprenant un matériau, ledit matériau présentant une énergie de surface de 25 dynes/cm ou moins.

**4.** Inhalateur selon la revendication 1, dans lequel ladite partie dudit bloc buse définissant ledit canal de sortie comprend une surface comprenant un matériau, ledit matériau ayant une surface uniforme ayant une valeur de 1 micron ou moins.

**5.** Inhalateur selon la revendication 1, dans lequel ladite partie dudit bloc buse définissant ledit canal de sortie comprend un matériau cristallin, métallique ou polymère.

**6.** Inhalateur selon la revendication 5, dans lequel ledit métal est choisi dans le groupe consistant en aluminium, or, nickel et acier inoxydable.

**7.** Inhalateur selon la revendication 5, dans lequel ledit matériau comprend un matériau polymère et ledit matériau polymère comprend du polyéthylène (PE), du polypropylène (PP), du polyméthylméthylacrylate (PMMA), du poly (chlorure de vinyle) (PVC), du poly(chlorure de vinylidène) (PVDC), du poly(fluorure de vinylidène) (PVDF), du polytrifluorochloréthylène (PCTFE), du polytétrafluoréthylène (PTFE), de l'éthylène-propylène fluoré (FEP), du perfluoroalcoxy (PFA), du polyamide (PA), du poly(éthylène téréphtalate) (PET), du polybutylène téréphtalate (PBT), du polyétherimide (PEI), du polyamide-imide (PAI), du polyimide (PI), de la polysulfone (PS), de la polyarylsulfone (PAS), de la polyéthersulfone (PES), du poly(phénylène sulfure) (PPS), du polyétheréthercétone (PEEK), du polydiméthylsiloxane (PDMS) et du polycarbonate (PC) ou des mélanges de ceux-ci.

**8.** Inhalateur selon la revendication 1, dans lequel ledit canal de sortie est conique, et la partie étroite comprend un rétrécissement dont le diamètre est inférieur à 0,3 mm et supérieur à environ 0,05 mm.

9. Inhalateur selon la revendication 1, dans lequel ladite partie étroite mesure 0,4 mm ou moins de long.

10. Inhalateur selon la revendication 2, dans lequel ladite plaque a une épaisseur de 1,0 millimètre ou moins.

11. Inhalateur selon la revendication 10, dans lequel ladite plaque a une épaisseur allant de 1,0 millimètre à 0,05 millimètre.

12. Inhalateur selon la revendication 1, dans lequel ledit trajet d'écoulement de fluide a un volume de 30 microlitres ou moins.

13. Inhalateur selon la revendication 1, dans lequel ladite chambre interne est tubulaire et conique, et le diamètre de la chambre diminue entre son admission et son évacuation.

14. Inhalateur selon la revendication 13, dans lequel ladite chambre interne s'incurve entre ladite admission et ladite évacuation.

15. Inhalateur selon la revendication 1, dans lequel le bloc buse définit une saillie, et ledit canal de sortie sort dudit bloc à partir de ladite saillie.

16. Inhalateur selon la revendication 15, dans lequel ladite saillie forme une pointe acérée où ledit canal émerge de ladite saillie.

17. Inhalateur selon la revendication 15, dans lequel ladite saillie comprend une pointe émoussée où ledit canal sort de ladite saillie.

18. Actionneur selon la revendication 1, dans lequel ladite chambre interne est lisse et conique et ne présente pas de caractéristiques de surface ou de cavités auxiliaires entre l'admission et l'évacuation, ce qui aurait créé un espace inutilisable dans celle-ci.

19. Inhalateur selon la revendication 1 ou 2, dans lequel ledit bloc buse possède entre 1 et 9 canaux de sortie.

20. Inhalateur selon la revendication 5, dans lequel ledit matériau cristallin est du silicium.

EP 1 292 395 B1

C

L

AB

MV

VS

N

E

NB

M

(PRIOR ART)

FiG·1

Fig.2B      Fig.2A      Fig.2c      Fig.2D

EP 1 292 395 B1

FiG.3A

FiG·3B

EP 1 292 395 B1

**Fig. 4B**

**Fig. 4A**

EP 1 292 395 B1

FIG. 5

23

FIG.6

FiG.7

Fig. 8

Drug deposition in expansion chamber for various actuators

Deposition as % of total dose

(7.73) (8.15) (9.29) (9.31) (10.05) (10.28) (15.50) (16.31) (16.60)

Actuator #

FiG.9

EP 1 292 395 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5031610 A
- US 4322037 A
- US 5497944 A
- US 4074861 A
- EP 0412524 A

### Non-patent literature cited in the description

- **LEWIS et al.** Effects of Actuator Orifice Diameter on Beclomethasone Dipropionate Delivery From a PMDI HFA Solution Formulation. *Poster Presented at Respiratory Drug Delivery VI,* 04 May 1998